# EUROPEAN PATENT APPLICATION

(11) **EP 2 255 624 A1**
(43) Date of publication of application: **01.12.2010**
(21) Application number: 09718879.1
(22) Date of filing: 26.02.2009
(51) Int. Cl.: A01N 43/16, A01N 65/18, A01P 3/00, A61K 8/49, A61K 8/97, A61K 31/352, A61K 36/47, A61P 31/04, A61Q 19/10

(54) **INHIBITOR FOR GROWTH OF BACTERIA OF GENUS LEGIONELLA, BATH AGENT AND CLEANING AGENT**

(30) Priority: 10.03.2008 JP 2008059780
(71) Applicant: POKKA CORPORATION, Nagoya-shi Aichi 460-8415 (JP)
(72) Inventor: GOTO, Takaki, Kitanagoya-shi Aichi 481-8515 (JP); FUKUMOTO, Syuichi, Kitanagoya-shi Aichi 481-8515 (JP)
(74) Representative: Melin Granberg, Linda
(86) International application number: PCT/JP2009/053607
(87) International publication number: WO 2009/113404

(57) **Abstract**

A growth inhibitor of bacteria of the genus *Legionella* contains as an active ingredient a *Macaranga tanarius* extract extracted from *Macaranga tanarius* with an extraction solvent including at least an organic solvent. Alternatively, the growth inhibitor of bacteria of the genus *Legionella* contains as an active ingredient at least one selected from nymphaeol-.A, nymphaeol-B, and nymphaeol-C. The growth inhibitor of bacteria of the genus *Legionella* is used by being blended to, for example, a bath agent or a cleaning agent.

## Description

### TECHNICAL FIELD

The present invention relates to a growth inhibitor of bacteria of the genus *Legionella,* and a bath agent and a cleaning agent containing the growth inhibitor.

### BACKGROUND ART

Bacteria of the genus *Legionella* are aerobic Gram-negative bacilli. It has been discovered that the bacteria are likely to grow in, for example, a bathing facility and a cooling tower. A *Legionella* infection is caused by inhalation of an aerosol containing bacteria of the genus *Legionella.* The *Legionella* infection includes *Legionella* pneumonia and Pontiac fever. *Legionella* pneumonia is known to be an infection with high mortality. Patent Document 1 discloses that an ingredient extracted from bamboo grass inhibits the growth of bacteria of the genus *Legionella.*

At the same time, as described in Patent Document 2, it is known that an extract of *Macaranga tanarius* (Oobagi), which belongs to the genus *Macaranga* of the family *Euphorbiaceae,* has an antimicrobial action. However, the action of the *Macaranga tanarius* extract on bacteria of the genus *Legionella* has not been clarified yet, and also Patent Document 2 does not describe it at all.
Patent Document 1: Japanese Laid-Open Patent Publication No. 2006-45077
Patent Document 2: Japanese Laid-Open Patent Publication No. 2007-45754

### DISCLOSURE OF THE INVENTION

The present invention is based on the fact that the inventors have found, as a result of their intensive studies, that an extract of *Macaranga tanarius* has an inhibitory action on the growth of bacteria of the genus *Legionella,* and an objective thereof is to provide a novel growth inhibitor of bacteria of the genus *Legionella,* and a bath agent and a cleaning agent containing the growth inhibitor.

In order to achieve the above-mentioned objective, a first aspect of the present invention provides a growth inhibitor of bacteria of the genus *Legionella* containing as an active ingredient a *Macaranga tanarius* extract extracted from *Macaranga tanarius* with an extraction solvent including at least an organic solvent.

A second aspect of the present invention provides a growth inhibitor of bacteria of the genus *Legionella* containing as an active ingredient at least one selected from nymphaeol-A, nymphaeol-B, and nymphaeol-C.

A third aspect of the present invention provides a bath agent containing the growth inhibitor of bacteria of the genus *Legionella* according to the above first or second aspect of the present invention.

A fourth aspect of the present invention provides a cleaning agent containing the growth inhibitor of bacteria of the genus *Legionella* according to the above first or second aspect of the present invention.

### BRIEF DESCRIPTION OF THE DRAWINGS

Fig. 1 is a chromatogram showing the results of high-performance liquid chromatography analysis of an extract of *Macaranga tanarius* according to Example 1; and
Fig. 2 is a chromatogram showing the results of high-performance liquid chromatography analysis of an extract of *Macaranga tanarius* according to Example 2.

### BEST MODE FOR CARRYING OUT THE INVENTION

An embodiment of the present invention will be described in detail below.

A growth inhibitor of bacteria of the genus *Legionella* according to the present embodiment contains as an active ingredient an Oobagi extract extracted from Oobagi with an extraction solvent including at least an organic solvent. Oobagi is also called *Macaranga tanarius* and is a dioecious broad-leaved evergreen tree belonging to the genus *Macaranga* of the family *Euphorbiaceae. Macaranga tanarius* grows, for example, in Southeast Asia, such as Okinawa (southern Japan), Taiwan, southern China, the Malay Peninsula, the Philippines, Malaysia, Indonesia, and Thailand, and in northern Australia. *Macaranga tanarius* grows significantly fast compared to other trees and can grow on degraded lands.

All the organs of *Macaranga tanarius* and constituents of each organ can be used as raw material to be subjected to extraction with the extraction solvent. The raw material for extraction may be a single organ of *Macaranga tanarius* or its constituents or may be a mixture of two or more organs of *Macaranga tanarius* or their constituents. In order to enhance the growth-inhibitory action of the resulting *Macaranga tanarius* extract on bacteria of the genus *Legionella,* it is preferred to use the raw material for extraction which includes fruit, seeds, flowers, roots, a trunk, the tip of a stem, a leaf blade, or an exudate (such as wax) of *Macaranga tanarius.* Since the tip of the stem includes a growth point of the stem and a leaf bud and is softer than the leaf blade, an efficient extraction procedure thereof is easy. Furthermore, the occupation ratios of the trunk, the roots, and the leaves to the entire *Macaranga tanarius* are high compared to those of other organs. Therefore, the use of leaf blade of *Macaranga tanarius* as a raw material for extraction is industrially advantageous from the standpoint of easiness of obtaining the raw material.

The raw material for extraction is subjected to an extraction procedure in the state when it is harvested, in the state that it is pulverized, crushed, or ground after the harvest, in the state that it is pulverized, crushed, or ground after the harvest and drying, or in the state that it is crushed, pulverized, or ground after the harvest and then is dried. In order to efficiently perform the extraction, the raw material for extraction is preferably crushed. The crushing of the raw material for extraction can be performed, for example, using a cutter, a shredder, or a crusher. The raw material for extraction can be pulverized using, for example, a mill, a crusher, or a grinder. The raw material for extraction can be ground using, for example, a kneader or a mortar.

The extraction solvent used for extracting a *Macaranga tanarius* extract from the raw material for extraction may be a solvent mixture of water and an organic solvent or may be an organic solvent such as lower alcohol, dimethyl sulfoxide, acetonitrile, acetone, ethyl acetate, hexane, glycerin, or propylene glycol. Examples of the lower alcohol that can be used include methanol, ethanol, propanol, isopropanol, and butanol. As the organic solvent, only one type of solvent may be used, or a mixture of a plurality of types of solvents may be used. When a solvent mixture of water and an organic solvent is used as the extraction solvent, the content of the organic solvent in the solvent mixture is preferably 50% by volume or more and more preferably 80% by volume or more. When the content of the organic solvent in a solvent mixture is 50% by volume or more, the active ingredient contained in *Macaranga tanarius* can be particularly efficiently extracted. The organic solvent is preferably lower alcohol and more preferably ethanol.

In the extraction solvent, for example, an organic salt, an inorganic salt, a buffer, an emulsifier, and dextrin may be dissolved.

The extraction is performed by immersing the raw material for extraction in the above extraction solvent for a predetermined time. In the extraction, according to need, for example, either stirring or heating or the both of them may be conducted for increasing the extraction efficiency. Furthermore, in order to minimize extraction of unnecessary impurities into the extraction solvent, prior to the extraction with the extraction solvent, the raw material for extraction may be prepared by being subjected to extraction with water or hot water and removing the extraction water in advance. The ingredient that is contained in *Macaranga tanarius* and presumably has an inhibitory action on the growth of bacteria of the genus *Legionella* is nymphaeols. The nymphaeols are water-insoluble. Impurities other than the nymphaeols are efficiently transferred to extraction water by boiling *Macaranga tanarius* with, for example, hot water and are thereby removed.

A *Macaranga tanarius* extract extracted from the raw material for extraction is subjected to solid liquid separation to separate and remove the residue of the raw material for extraction. The solid liquid separation is performed, for example, by a known method such as filtration or centrifugation. The *Macaranga tanarius* extract in a liquid form after the solid liquid separation may be concentrated according to need.

A *Macaranga tanarius* extract in a solid form can be obtained by removing the extraction solvent contained in the *Macaranga tanarius* extract in the liquid form, according to need. The removal of the extraction solvent from the *Macaranga tanarius* extract in the liquid form may be performed, for example, by heating under reduced pressure or by lyophilization.

The *Macaranga tanarius* extract extracted from *Macaranga tanarius* with an extraction solvent including at least an organic solvent contains at least one selected from nymphaeol-A (also known as 5,7,3',4'-tetrahydroxy-6-geranylflavanone), nymphaeol-B (also known as 5,7,3',4'-tetrahydroxy-2'-geranylflavanone), and nymphaeol-C (also known as 5,7,3',4'-tetrahydroxy-6-(3"',3"'-dimethylallyl)-2'-geranylflavanone). A main ingredient of the *Macaranga tanarius* extract is at least one selected from nymphaeol-A, nymphaeol-B, and nymphaeol-C, that is, nymphaeols, and the nymphaeols presumably have an inhibitory action on the growth of bacteria of the genus *Legionella.*

The *Macaranga tanarius* extract further contains propolin A (also known as 5,7,3',4'-tetrahydroxy-2'-(7"-hydroxy-3",7"-dimethyl-2"-octenyl)-flavanone). Furthermore, the *Macaranga tanarius* extract contains as minor ingredients, for example, 5,7,3',4'-tetrahydroxy-5'-geranylflavanone (also known as isonymphaeol-B), 5,7,3',4'-tetrahydroxy-5'-(7"-hydroxy-3",7"-dimethyl-2"-octenyl)-flavanone,5,7,3',4'-tetrahydroxy-6-(7"-hydroxy-3",7"-dimethyl-2"-octenyl)-flavanone, 5,7,4'-trihydroxy-3'-(7"-hydroxy-3",7"-dimethyl-2"-octenyl)-flavanone, and 5,7,4'-trihydroxy-3'-geranylflavanone.

Among extract solutions each extracted from portions of *Macaranga tanarius,* an extract solution extracted from flowers, seeds, and fruit (containing wax) particularly contains high concentrations of nymphaeol-A, B, and C and isonymphaeol-B.

The growth inhibitor of bacteria of the genus *Legionella* may contain a component other than the *Macaranga tanarius* extract as long as the inhibitory action on the growth of bacteria of the genus *Legionella* is not impaired. Examples of the component that can be contained in the growth inhibitor of bacteria of the genus *Legionella,* in addition to the *Macaranga tanarius* extract, include an excipient, a base, an emulsifier, a stabilizer, and a flavoring.

The growth inhibitor of bacteria of the genus *Legionella* may be in a liquid form or in a solid form. The dosage form of the growth inhibitor of bacteria of the genus *Legionella* is not particularly limited and may be, for example, a powder, a dust, a granule, a tablet, a capsule, a pill, or a liquid. The growth inhibitor of bacteria of the genus *Legionella* prepared in a solid form is used, for example, by being supported on a filter that is set in a pipe for drainage or water circulation and a bacteria removal facility.

For example, a surfactant, a chelating agent, an emulsifier, or ethanol can be added to the growth inhibitor of bacteria of the genus *Legionella* to improve the water dispersibility and water solubility of the *Macaranga tanarius* extract.

Inhibition of the growth of bacteria of the genus *Legionella* is extremely important in order to prevent a *Legionella* infection such as *Legionella* pneumonia and Pontiac fever caused by inhalation of an aerosol containing bacteria of the genus *Legionella.* The growth inhibitor of bacteria of the genus *Legionella* is useful in preventing human infection with bacteria of the genus *Legionella* through inhibition of the growth of bacterial of the genus *Legionella.*

While *Legionella pneumophila* is known as a representative bacterium of the genus *Legionella,* several tens of species of bacteria of the genus *Legionella* have been identified at present, and all of them are considered to possibly cause a *Legionella* infection.

The growth inhibitor of bacteria of the genus *Legionella* can be applied to a site where the growth of bacterial of the genus *Legionella* is expected without any particular limitation. Specifically, the growth inhibitor of bacteria of the genus *Legionella* is added, for example, to water used in a bathing facility, a water and hot-water supply facility, a cooling tower, a humidifier, a waterscape facility, and a thermal storage tank. Infections caused by bacterial of the genus *Legionel*/*a* is likely to occur particularly in the elderly, neonates, and infants and young children, who have low resistance. Since the active ingredient of the growth inhibitor of bacteria of the genus *Legionella* of the present embodiment is the *Macaranga tanarius* extract, which is originated from a natural substance, the growth inhibitor can be safely used in a medical facility and a welfare facility for the elderly.

The growth inhibitor of bacteria of the genus *Legionella* can be used as, for example, a bath agent, a cleaning agent, a deodorant, and a fragrance. Since the growth inhibitor is easily applicable to such a site that could be a source of generation of the bacteria as described above, it is preferably used as a bath agent or a cleaning agent. It is desirably used in such a way that the total concentration of nymphaeol-A, nymphaeol-B, and nymphaeol-C, that is, the concentration of nymphaeols, in a site that could be a source of generation of the bacteria of the genus *Legionella* is preferably 15 ppm or more, and more preferably 20 ppm or more. When the concentration of nymphaeols is 15 ppm or more, an inhibitory action on the growth of bacteria of the genus *Legionella* is particularly well exerted.

The bath agent of the present embodiment contains the above-mentioned growth inhibitor of bacteria of the genus *Legionella.* The agent form of the bath agent can be, for example, a solid, a liquid, and a powder. The bath agent contains at least one selected from, for example, a water-soluble inorganic salt, a colorant, a fragrance, a humectant, a pH regulator, a vitamin, a herbal medicine, an organic salt, and an enzyme, in addition to the above-mentioned growth inhibitor of bacteria of the genus *Legionella.* Examples of the water-soluble inorganic salt include a carbonate such as sodium carbonate and sodium bicarbonate, a silicate such as sodium metasilicate, a phosphate such as sodium orthophosphate, a sulfate such as sodium sulfate and magnesium sulfate, and a chloride such as sodium chloride, calcium chloride, and ammonium chloride.

By using such a bath agent by mixing it into a bath liquid in a bathing facility, the growth of bacteria of the genus *Legionella* can be inhibited in a bathtub, a pipe circulating the bath liquid into the bathtub, a filter connected to the pipe, and a heat exchanger while the bathing facility is in operation. Therefore, the bath agent of the present embodiment is suitable for preventing human infection by bacteria of the genus *Legionella* in various bathing facilities. The content of the *Macaranga tanarius* extract in the bath agent can be appropriately set according to the amount of the bath agent to be mixed in a bath liquid. The bath agent is desirably used in such a way that the total concentration of nymphaeol-A, nymphaeol-B, and nymphaeol-C, that is, the concentration of nymphaeols, in a bath liquid is preferably 15 ppm or more, and more preferably 20 ppm or more. When the concentration of nymphaeols in a bath liquid is 15 ppm or more, an inhibitory action on the growth of bacteria of the genus *Legionella* is particularly well exerted.

The cleaning agent of the present embodiment contains the above-mentioned growth inhibitor of bacteria of the genus *Legionella.* The agent form of the cleaning agent can be, for example, a solid, a liquid, and a powder. The cleaning agent contains at least one selected from, for example, a surfactant, a chelating agent, and a pH regulator, in addition to the above-mentioned growth inhibitor of bacteria of the genus *Legionella.* The cleaning agent may further contain a chlorine-based or an oxygen-based disinfectant according to need.

The cleaning agent is used, for example, by being periodically contacted with a site that could be a source of generation of bacterial of the genus *Legionella* for a predetermined time. In more detail, a site that could be a source of generation of bacterial of the genus *Legionella* may be immersed in a diluted liquid of the cleaning agent, which is obtained by appropriately diluting the cleaning agent. Alternatively, the cleaning agent may be directly sprayed to a site that could be a source of generation of bacterial of the genus *Legionella.* The content of the *Macaranga tanarius* extract in the cleaning agent can be appropriately set according to the application form of the cleaning agent The cleaning agent is desirably used in such a way that the total concentration of nymphaeols in a site that could be a source of generation of bacterial of the genus *Legionella* is preferably 15 ppm or more, and more preferably 20 ppm or more. When the concentration of nymphaeols is 15 ppm or more, an inhibitory action on the growth of bacteria of the genus *Legionella* is particularly well exerted.

According to the present embodiment, the following advantageous effects are achieved.

The growth inhibitor of bacteria of the genus *Legionella* of the present embodiment is a novel growth inhibitor of bacteria of the genus *Legionella* containing the *Macaranga tanarius* extract as an active ingredient. The growth inhibitor is useful in preventing infection by bacteria of the genus *Legionella* through inhibition of the growth of bacterial of the genus *Legionella.*

In addition, since *Macaranga tanarius* grows significantly fast compared to other trees and can grow on degraded lands, the cultivation does not take much effort. Furthermore, since the *Macaranga tanarius* extract is originated from a plant, it is highly safe. Therefore, the growth inhibitor of bacteria of the genus *Legionella* of the present embodiment is also excellent in stable supply of raw material, productivity, and safety.

Since the bath agent and the cleaning agent of the present embodiment contain the above-mentioned growth inhibitor of bacteria of the genus *Legionella,* they have an inhibitory action on the growth of bacteria of the genus *Legionella.*

The above-described embodiment may be modified as follows.

The growth inhibitor of bacteria of the genus *Legionella* of the above embodiment may contain at least one selected from nymphaeol-A, nymphaeol-B, and nymphaeol-C that are not originated from *Macaranga tanarius* extracts, as an active ingredient, instead of the *Macaranga tanarius* extract or in addition to the *Macaranga tanarius* extract. Nymphaeol-A, nymphaeol-B, and nymphaeol-C that are not originated from *Macaranga tanarius* extracts can be obtained by, for example, chemical synthesis.

Bacteria of the genus *Legionella* exist in a natural environment such as soil. Therefore, there is a risk that bacteria of the genus *Legionella* might grow in soil or fertilizer for gardening that is kept in a sealed bag made of resin. In view of the above, with the aim of preventing human infection by bacteria of the genus *Legionella* via soil or fertilizer for gardening, the growth inhibitor of bacteria of the genus *Legionella* of the above-mentioned embodiment may be used by being added to soil or fertilizer for gardening. Since the active ingredient of the growth inhibitor of bacteria of the genus *Legionella* is the *Macaranga tanarius* extract, which is originated from a natural substance, when the soil or the fertilizer containing the growth inhibitor of bacteria of the genus *Legionella* is used to cultivate a garden plant, the growth of the plant is hardly inhibited.

Next, the present invention will be further specifically described with reference to examples.

### Example 1

### <Preparation 1 of Macaranga tanarius extract>

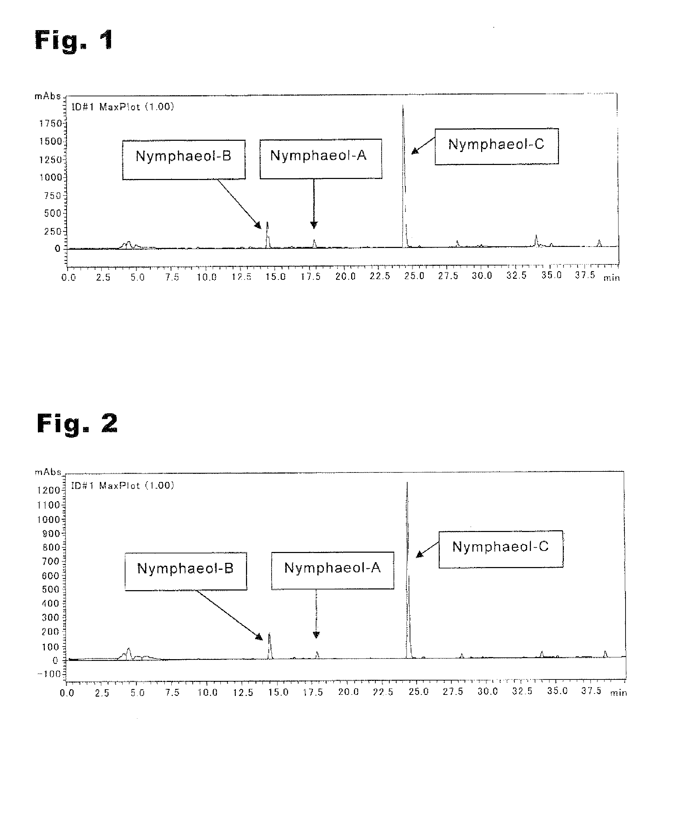

Frozen raw leaves of *Macaranga tanarius* harvested in Okinawa were thawed, and the leaves were cut into small pieces with scissors. Thirty grams of the cut raw leaves were immersed in 10D mL of a solvent mixture consisting of 90 parts by volume of ethanol and 10 parts by volume of water and left standing at room temperature for two weeks, followed by filtration to yield the filtrate as a *Macaranga tanarius* extract solution. The *Macaranga tanarius* extract solution was lyophilized to prepare a *Macaranga tanarius* extract that was a powder of the solid content contained in the *Macaranga tanarius* extract solution. The total concentration of nymphaeol-A, nymphaeol-B, and nymphaeol-C, that is, the concentration of nymphaeols, in the *Macaranga tanarius* extract in the powder form was 50% by mass when calculated from the chromatogram shown in Fig. 1 obtained by analyzing the *Macaranga tanarius* extract under the following HPLC conditions.

### HPLC conditions

System: PDA-HPLC system (Shimadzu Corp.), LC10ADvp series, UV: SPD-10Avp, PDA: SPD-M10Avp,
Column: Luna C18 (2 × 250 mm) (Shimadzu GLC),
Solvent: A: water (5% acetic acid), B: acetonitrile (5% acetic acid)
Dissolution condition:
0 to 20 minutes
(gradient dissolution: A:B = 80:20 → A:B = 30:70)
20 to 50 minutes
(gradient dissolution: A:B = 30:70 → A:B = 0:100)
50 to 60 minutes (A:B = 0:100)
60 to 75 minutes (A:B = 80:20)
Flow rate: 0.2 mL/min
PDA detection: UV from 190 to 370 nm
UV detection: UV 287 nm
Injection amount: 20 µL
Temperature: 40°C

### <Test of antimicrobial activity of Macaranga tanarius extract on bacteria of genus Legionella>

### Preculture

A strain of bacteria of the genus *Legionella, Legionella* pneumophila (GIFU9134), was cultured at 37°C for four days using a buffered charcoal yeast extract (BCYE) medium supplemented with L-cysteine. Then, the bacteria thus obtained were suspended in a phosphate buffer to prepare a bacterial liquid for inoculation having a bacterial count of 10⁶ to 10⁷ CFU/mL.

### Preparation of sample liquid

The above-mentioned *Macaranga tanarius* extract in a powder form was mixed in 99.5 V/V% ethanol to prepare a sample liquid having a concentration of the *Macaranga tanarius* extract of 100 × 10³ ppm. Further, by diluting the sample liquid with 99.5 V/V% ethanol, sample liquids in doubling-dilution series, that is, sample liquids having respective concentrations of the *Macaranga tanarius* extract of 50 × 10³ ppm, 25 × 10³ ppm, 12.5 × 10³ ppm, 6.25 × 10³ ppm, 3.13 × 10³ ppm, and 1.56 × 10³ ppm, were also prepared.

### Preparation of plate medium for sensitivity measurement

To 99% by mass of B-SYEα media, which had been kept warm at 50 to 60°C after sterilization, 1% by mass of each sample liquid was added and thoroughly mixed. The mixtures were dispensed into petri dishes and solidified. Accordingly, plate media for sensitivity measurement having respective concentrations of the *Macaranga tanarius* extract of 1,000 ppm, 500 ppm, 250 ppm, 125 ppm, 62.5 ppm, 31.3 ppm, and 15.6 ppm were prepared. The plate media thus prepared were dried in a clean bench. B-SYEα media is composed of 1.5 g of starch, 1.0 g of yeast extract, 1.3 g of agar, 1 vial of *Legionella* BSYEα Growth Supplement (OXOID), and 100 mL of purified water.

### Inoculation and culturing of bacteria of genus Legionella

The bacterial liquid for inoculation was streaked on the plate media for sensitivity measurement using a loop having an inner diameter of 1 mm. Subsequently, the bacterial strain on each plate medium was cultured at 37°C for three days.

### Assessment and results

Observation of the plate media after culturing showed that the growth of bacteria of the genus *Legionella* was inhibited in the plate media having a concentration of the *Macaranga tanarius* extract of 31.3 ppm or more. In other words, the minimum inhibitory concentration (MIC) of the *Macaranga tanarius* extract of Example 1 against bacteria of the genus *Legionella* was 31.3 ppm. As described above, since the *Macaranga tanarius* extract of Example 1 contains 50% by mass of nymphaeols, it is speculated from the above results that the concentration of nymphaeols in practical application is preferably 15.6 ppm or more.

### Example 2

### <Preparation 2 of Macaranga tanarius extract>

Thirty grams of cut raw leaves of *Macaranga tanarius* were immersed in 95°C water for 30 minutes. The water was removed by filtration, and the remaining leaves were immersed in 100% ethanol for 3 days, followed by filtration to yield the filtrate as a *Macaranga tanarius* extract solution. The *Macaranga tanarius* extract solution was lyophilized to prepare a *Macaranga tanarius* extract that was a powder of the solid content contained in the *Macaranga tanarius* extract solution. The total concentration of nymphaeol-A, nymphaeol-B, and nymphaeol-C, that is, the concentration of nymphaeols, in the *Macaranga tanarius* extract in the powder form was 40% by mass when calculated from the chromatogram shown in Fig. 2 obtained by analyzing the *Macaranga tanarius* extract under the above-mentioned HPLC conditions.

### <Test of antimicrobial activity of Macaranga tanarius extract on bacteria of genus Legionella>

An antimicrobial activity test was performed as in Example 1. Regarding the *Macaranga tanarius* extract prepared in Example 2, similar results as those of the *Macaranga tanarius* extract prepared in Example 1 were obtained to show that the antimicrobial activities of the *Macaranga tanarius* extract prepared in Example 1 and the *Macaranga tanarius* extract prepared in Example 2 were similar to each other.

## Claims

1. A growth inhibitor of bacteria of the genus Legionella **characterized by** comprising as an active ingredient a *Macaranga tanarius* extract extracted from *Macaranga tanarius* with an extraction solvent including at least an organic solvent.

2. A growth inhibitor of bacteria of the genus *Legionella* **characterized by** comprising as an active ingredient at least one selected from nymphaeol-A, nymphaeol-B, and nymphaeol-C.

3. The growth inhibitor of bacteria of the genus *Legionella* according to claim 2, wherein the at least one selected from nymphaeol-A, nymphaeol-B, and nymphaeol-C is originated from an extract of *Macaranga tanarius.*

4. A bath agent **characterized by** comprising the growth inhibitor of bacteria of the genus *Legionella* according to any one of claims 1 to 3.

5. A cleaning agent **characterized by** comprising the growth inhibitor of bacteria of the genus *Legionella* according to any one of claims 1 to 3.
